(19) Europäisches Patentamt
European Patent Office
Office européen des brevets

(11) Veröffentlichungsnummer: **0 380 043 B1**

# (12) EUROPÄISCHE PATENTSCHRIFT

(45) Veröffentlichungstag der Patentschrift: **22.12.93**

(51) Int. Cl.⁵: **C07C 69/712**, C07C 67/31

(21) Anmeldenummer: **90101267.4**

(22) Anmeldetag: **23.01.90**

## (54) Verfahren zur Herstellung von D(+)-2-(4-Acetylphenoxy)- propionsäureestern.

(30) Priorität: **27.01.89 DE 3902372**

(43) Veröffentlichungstag der Anmeldung:
**01.08.90 Patentblatt 90/31**

(45) Bekanntmachung des Hinweises auf die Patenterteilung:
**22.12.93 Patentblatt 93/51**

(84) Benannte Vertragsstaaten:
**CH DE FR GB IT LI**

(56) Entgegenhaltungen:
**EP-A- 0 334 596**
**US-A- 4 532 328**

**CHEMICAL ABSTRACTS, Band 108, 1988, Zusammenfassung Nr. 5692k, Columbus, Ohio, US; & JP-A-62 178 543**

(73) Patentinhaber: **HOECHST AKTIENGESELLSCHAFT**

**D-65926 Frankfurt(DE)**

(72) Erfinder: **Schlegel, Günter, Dr.**
**Feldbergstrasse 18**
**D-6237 Liederbach(DE)**
Erfinder: **Mildenberger, Hilmar, Dr.**
**Fasanenstrasse 24**
**D-6233 Kelkheim (Taunus)(DE)**

EP 0 380 043 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Optisch aktive 2-(4-Acetylphenoxy)-propionsäureester sind wertvolle Vorstufen für die Herstellung von 2-(4-Hydroxyphenoxy)propionsäurederivaten, die ihrerseits als Ausgangsverbindungen für herbizide (Hetero)aryloxyphenoxypropionsäurederivaten Verwendung finden, z.B. für die Herstellung von Fenoxaprop-ethyl; siehe DE-A 26 40 730 (US-A 4,130,413) und EP-A 2800 (US-A 4,531,969). Die Titelverbindungen sind aus JP-A 62 178543 bekannt. Das dort angegebene Verfahren (Umsetzung von 4-Hydroxyacetophenon mit L-Milchsäureester) wird bevorzugt bei 80-100 °C ausgeführt. Dabei wird jedoch das entstehende D-Enantiomere des Phenoxypropionats zu einem erheblichen Teil racemisiert. Dies hätte bei der weiteren Umsetzung die Bildung von herbizid unwirksamen L-(Hetero)aryloxyphenoxypropionsäurederivaten zur Folge. Da es aus ökonomischen und ökologischen Gründen wünschenswert ist, den herbiziden Wirkstoff möglichst enantiomerenrein einzusetzen, muß die L-Komponente aus dem Vorprodukt abgetrennt werden, was nur mit erheblichem Aufwand möglich ist.

Überraschenderweise wurde nun gefunden, daß die Bildung des unerwünschten L-Enantiomeren fast völlig unterbleibt, wenn man bei der in JP-A 62 178543 beschriebenen Reaktion Umsetzungstemperaturen nicht über 50 °C, d.h. weit unterhalb des angegebenen Vorzugsbereichs, anwendet.

Gegenstand der Erfindung ist somit ein Verfahren zur Herstellung von D(+)-2-(4-Acetylphenoxy)-propionsäureester der Formel (I)

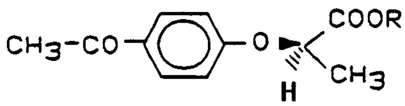

(I)

durch Umsetzung von 4-Hydroxyacetophenon mit L-Milchsäurederivaten der Formel (II),

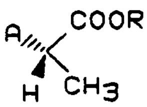

(II)

wobei in den angegebenen Formeln
R ein Alkyl mit 1 bis 4 C-Atomen und
A Halogen oder eine Sulfonyloxygruppe
bedeutet, dadurch gekennzeichnet, daß man die Umsetzung bei Temperaturen von höchstens 50 °C in basischem Medium ausführt.

Für R kommen Methyl, Ethyl, n- und i-Propyl, n-, i-, t- und 2-Butyl in Frage, insbesondere Methyl und Ethyl.

Als Halogen kommt bevorzugt Chlor, Brom oder Jod infrage; Beispiele für die Sulfonyloxy-Gruppe sind Methansulfonyloxy, Trifluormethansulfonyloxy, Benzolsulfonyloxy und o-, m- und p-Toluolsulfonyloxy. Besonders bevorzugte Abgangsgruppen A sind Chlor, Brom, die Methansulfonyloxy- oder die p-Toluolsulfonyloxy-Gruppe.

Die Reaktion wird bevorzugt in einem unter den Reaktionsbedingungen inerten, vorzugsweise organischen Lösungsmittel oder -gemisch ausgeführt. Beispiele für geeignete Lösungsmittel sind Toluol oder Xylol in Verbindung mit Phasentransferkatalysatoren wie Tetraalkylammoniumsalzen oder Kronenethern, Polyethylenglycol oder dipolar-aprotische Solventien wie Dimethylformamid, N-Methylpyrrolidon, Dimethylsulfoxid, Sulfolan oder Acetonitril.

Man setzt dem Reaktionsgemisch entweder eine schwache Hilfsbase zu oder überführt das 4-Hydroxyacetophenon zunächst ins Alkali-, Erdalkali- oder Ammoniumsalz, bevor es mit Verbindungen der Formel (II) zur Umsetzung gebracht wird. Als Hilfsbase kommen beispielsweise Alkalimetallcarbonate und -hydrogencarbonate z.B. Natrium- oder Kaliumhydrogencarbonat, Natrium- oder Kaliumcarbonat in Frage. Bewährt hat sich ein Zusatz von 1-10 % Cäsiumcarbonat.

Die Reaktionstemperatur beträgt vorzugsweise 20-50 °C, abhängig vom verwendeten Lösungsmittel, der Gruppierung A und der Hilfsbase. Die Reaktionsdauer beträgt je nach Temperatur, Lösungsmittel, Hilfsbase und evtl. Phasentransferkatalysator 1-20, bevorzugt 2-12 Std.

**Herstellungsbeispiele**

1) 136 g (1,0 mol) 4-Hydroxyacetophenon werden zu einer Suspension von 138 g (1,0 mol) Kaliumcarbonat in 1 l Dimethylsulfoxid gegeben und innerhalb von 30 min. 122,5 g (1 mol) L-Methyl-2-chlorpropionat hinzugefügt. Nach 6-stündigem Rühren bei Raumtemperatur wird mit 5 g gemahlenem Kaliumcarbonat versetzt und weitere 6 Std. bei Raumtemperatur gerührt.

Man gießt auf 1,5 l Eiswasser und extrahiert mit 300 ml Ether. Nach Trocknen und Abdampfen der Etherphase bleibt D(+)-Methyl-2-(4-acetylphenoxy)propionat als bernsteinfarbenes Wachs zurück, das langsam durchkristallisiert. Ausb.: 209 g (94 % d.Th.); Fp.: 42-44 °C; $[\alpha]_D^{23}$ = + 45,2 ° (c = 1,00 in $CHCl_3$); die gaschromatographisch bestimmte Reinheit beträgt > 98,5 %; Enantiomerenreinheit (über chirale HPLC): D(+) 93 %, L(-) 7 %.

2a) 77,4 ml (1 mol) Methansulfonsäurechlorid werden zu einer Lösung von 124,8 ml (1,1 mol) S-Milchsäureethylester (94 % ee) in 750 ml Tetrahydrofuran gegeben und bei 20-22 °C mit 166,4 ml (1,2 mol) Triethylamin über 1,5 Std. versetzt. Man erhitzt innerhalb von 2 Std. auf 60 °C, rührt 2 weitere Std. bei dieser Temperatur und gibt auf eine Mischung aus Eis und konz. Salzsäure. Extraktion mit Ether, Trocknen und Einengen liefert 192,7 g (98 % d.Th.) L-Ethyl-2-methansulfonyloxypropionat als zähes Öl (-$[\alpha]_D^{23}$ = -52,04 (c = 1,38 in $CHCl_3$)). Es kann als Rohprodukt für die folgende Umsetzung benutzt werden.

2b) 98 g (0,5 mol) des unter 2a) beschriebenen L-Ethyl-2-methansulfonyloxy-propionats werden mit 68 g (0,5 mol) 4-Hydroxyacetophenon und 69,1 g (0,5 mol) Kaliumcarbonat in 400 ml DMSO 30 min. bei Raumtemperatur und 6 Std. bei 37 °C gerührt. Man gießt das Produkt auf eine Eis/Salzsäuremischung, extrahiert mit Ether und erhält nach Einengen des getrockneten Extrakts 113,5 g (96 %) D(+)-Ethyl-2-(4-acetylphenoxy)-propionat als gelbes zähflüssiges Öl; $[\alpha]_D^{23}$ = +44,15 ° (c = 1 in $CHCl_3$); Reinheit nach GC: 97 %; Enantiomerenreinheit (über chirale HPLC): D(+) 95,5 %, L(-) 4,5 %.

Die Einhaltung einer Reaktionstemperatur bis 50 °C ist wesentlich für eine hohe optische Ausbeute. Wie Tabelle I deutlich macht, führt eine Temperaturerhöhung zu merklicher Racemisierung der Komponente II (A = Cl, R = $CH_3$). Mit dieser Racemisierung verbunden ist eine geringere optische Reinheit des Ethers I (R = $CH_3$), der nach dem Herstellungsbeispiel 1) bei verschiedenen Temperaturen synthetisiert wurde (Tabelle II).

**Tabelle I:**

Racemisierung von S-Methyl-2-chlorpropionat in DMSO in Gegenwart molarer Mengen Kaliumcarbonat ($\alpha_0$ = Drehwert vor, $\alpha$ = Drehwert nach Behandlung)

| Dauer der Einwirkung | Temperatur | Racemisierung $(1-\alpha/\alpha_0) \cdot 100$ % |
|---|---|---|
| 4 h | 23°C | < 2 %* |
| 4 h | 40°C | 5 % |
| 4 h | 60°C | 60 % |
| 1 h | 110°C | 100 % |

* $\alpha \doteq \alpha_0$ im Rahmen der Meßgenauigkeit

**Tabelle II:**

Drehwert von Verbindung I (R = $CH_3$) in Abhängigkeit von der Bildungstemperatur

| Temperatur | Drehwert $[\alpha]_D^{23}$ |
|---|---|
| 23°C | 45,2° |
| 50°C | 41,4° |
| 60°C | 39,8° |

Die bei Temperaturen von höchstens 50 °C dargestellten D(+)-2-(4-Acetylphenoxy)-propionsäureester können durch die Baeyer-Villiger-Reaktion unter Erhalt der optischen Reinheit in R(+)-2-(4-Hydroxyphenoxy)-propionsäureester überführt werden (s. z.B. J. Am. Chem. Soc. 72, 878 (1950), J 62-178543). Diese reagieren mit halogensubstituierten Heterocyclen zu den wirksamen Enantiomeren bekannter Grasherbizide wie z.B. Haloxyfop-methyl, Quizalofop-ethyl, Fenoxaprop-ethyl oder Fluazifop-butyl.

## Patentansprüche

1. Verfahren zur Herstellung von D(+)-2-(4-Acetylphenoxy)-propionsäureester der Formel (I)

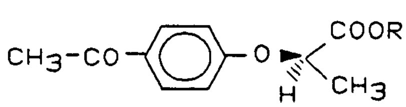

(I)

durch Umsetzung von 4-Hydroxyacetophenon mit L-Milchsäurederivaten der Formel (II),

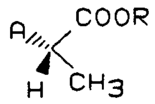

( I I )

wobei in den angegebenen Formeln
R ein Alkyl mit 1 bis 4 C-Atomen und
A Halogen oder eine Sulfonyloxygruppe
bedeutet, dadurch gekennzeichnet, daß man die Umsetzung bei Temperaturen von höchstens 50 °C in basischem Medium ausführt.

2. Verfahren nach Anspruch 1, dadurch gekennzeichnet, daß man die Umsetzung bei 20 bis 50 °C durchführt.

3. Verfahren nach Anspruch 1 oder 2, dadurch gekennzeichnet, daß man die Umsetzung in einem unter den Reaktionsbedingungen inerten organischen Lösungsmittel oder -gemisch durchführt.

4. Verfahren nach einem oder mehreren der Ansprüche 1 bis 3, dadurch gekennzeichnet, daß man als Hilfsbase Alkalimetallcarbonate oder Alkalimetallhydrogencarbonate einsetzt.

5. Verfahren nach einem oder mehreren der Ansprüche 1 bis 4, dadurch gekennzeichnet, daß A Chlor, Brom, Jod, Methansulfonyloxy, Trifluormethansulfonyloxy, Benzolsulfonyloxy oder Toluolsulfonyloxy bedeutet.

6. Verfahren nach einem oder mehreren der Ansprüche 1 bis 5, dadurch gekennzeichnet, daß man die Umsetzung in 1 bis 20 Std. durchführt.

## Claims

1. A process for the preparation of a D(+)-2-(4-acetylphenoxy)-propionic ester of the formula (I)

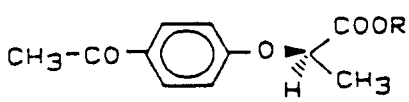

(I)

by reacting 4-hydroxyacetophenone with L-lactic acid derivatives of the formula (II),

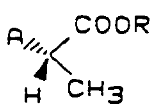

(II)

where, in the formulae indicated,
R is alkyl having 1 to 4 carbon atoms and
A is halogen or a sulfonyloxy group,
which comprises carrying out the reaction in a basic medium at temperatures of not more than 50 °C.

2. The process as claimed in claim 1, wherein the reaction is carried out at 20 to 50 °C.

3. The process as claimed in claim 1 or 2, wherein the reaction is carried out in an organic solvent or solvent mixture which is inert under the reaction conditions.

4. The process as claimed in one or more of claims 1 to 3, wherein an alkali metal carbonate or an alkali metal hydrogen carbonate is employed as auxiliary base.

5. The process as claimed in one or more of claims 1 to 4, wherein A is chlorine, bromine, iodine, methanesulfonyloxy, trifluoromethanesulfonyloxy, benzenesulfonyloxy or toluenesulfonyloxy.

6. The process as claimed in one or more of claims 1 to 5, wherein the reaction is carried out over 1 to 20 hours.

**Revendications**

1. Procédé de préparation d'esters de l'acide D(+)-2-(4-acétylphénoxy)-propionique de formule (I),

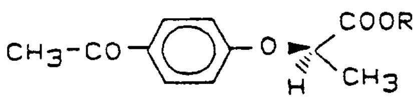

(I)

par réaction de la 4-hydroxyacétophénone avec des dérivés de l'acide L-lactique de formule (II),

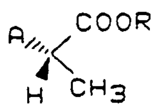

(II)

et, dans les formules indiquées ,

R représente un reste alkyle ayant 1 à 4 atomes de carbone, et

A représente un atome d'halogène ou un groupe sulfonyloxy,

procédé caractérisé en ce qu'on effectue la réaction à des températures de 50°C au maximum dans un milieu basique.

2. Procédé selon la revendication 1, caractérisé en ce qu'on conduit la réaction entre 20 et 50°C.

3. Procédé selon la revendication 1 ou 2, caractérisé en ce qu'on conduit la réaction dans un solvant ou un mélange de solvants organique(s) inerte(s) dans les conditions de réaction.

4. Procédé selon une ou plusieurs des revendications 1 à 3, caractérisé en ce qu'on utilise comme base auxiliaire des carbonates de métaux alcalins ou des hydrogénocarbonates de métaux alcalins.

5. Procédé selon une ou plusieurs des revendications 1 à 4, caractérisé en ce que A représente un atome de chlore, de brome ou d'iode, un groupe méthane sulfonyloxy, trifluorométhane sulfonyloxy, benzène sulfonyloxy ou toluène sulfonyloxy

6. Procédé selon une ou plusieurs des revendications 1 à 5, caractérisé en ce qu'on conduit la réaction en 1 à 20 heures.